# EUROPEAN PATENT SPECIFICATION

(11) **EP 1 267 990 B1**
(45) Date of publication and mention of the grant of the patent: **29.07.2015**
(21) Application number: 01924384.9
(22) Date of filing: 27.03.2001
(51) Int. Cl.: G01N 33/49, A61M 1/02, A61M 1/36

(54) **SYSTEMS AND METHODS FOR COLLECTING LEUKOCYTE-REDUCED BLOOD COMPONENTS, INCLUDING PLASMA THAT IS FREE OR VIRTUALLY FREE OF CELLULAR BLOOD SPECIES**
ANORDNUNGEN UND VERFAHREN ZUR SAMMLUNG VON BLUTKOMPONENTEN MIT REDUZIERTEM LEUKOZYTENANTEIL BEINHALTEND PLASMA, DAS (PRAKTISCHE) FREI VON ZELLULAREN BLUTANTEN IST
SYSTÈMES ET PROCÉDÉS POUR COLLECTER DES COMPOSANTS SANGUINS SANS LEUCOCYTES, COMPRENANT LE PLASMA EXEMPT OU PRATIQUEMENT EXEMPT D'ESPÈCES SANGUINES CELLULAIRES

(30) Priority: 31.03.2000 US 540935; 22.11.2000 US 252870 P
(43) Date of publication of application: 02.01.2003
(73) Proprietor: Fenwal, Inc., Lake Zurich, IL 60047 (US)
(72) Inventor: LYNN, Daniel, R., Spring Grove, IL 60081 (US); VAN HEEMS, Phillippe, 36400 LaChatre (FR); MUI, Tat, Chicago, IL 60660 (US); BERNES, Jean-Claude, 4317 Faimes (BE); DE VOS, Robert, 1428 Lillois-Witterzee (BE); MATHIAS, Jean-Marie, 1428 Lillois (BE)
(74) Representative: Geary, Stephen
(86) International application number: PCT/US2001/009865
(87) International publication number: WO 2001/074158

(56) References cited:
- EP-A- 0 976 413
- US-A- 5 180 504
- US-A- 5 527 472
- US-A- 5 738 796
- US-A- 5 804 079
- US-A- 5 836 934
- US-A- 6 051 147

## Description

### Field of the Invention

The invention generally relates to the processing of whole blood and its components for storage, fractionation, and transfusion.

### Background of the Invention

With the coming of blood component therapy, most whole blood collected today is separated into its clinically proven components for storage and administration. The clinically proven components of whole blood include, e.g., red blood cells, which can be used to treat chronic anemia; plasma, which can be used as a blood volume expander or which can be fractionated to obtain Clotting Factor VIII-rich cryoprecipitate for the treatment of hemophilia; and concentrations of platelets, used to control thrombocytopenic bleeding.

Along with the growing demand for these blood components, there is also a growing expectation for purity of the blood product. Before storing blood components such as red blood cells or platelets for later transfusion, it is believed to be desirable to minimize the presence of impurities or other materials that may cause undesired side effects in the recipient. Because of possible reactions, it is generally considered desirable to remove substantially all the leukocytes from such blood components before storage, or at least before transfusion.

It is also believed beneficial that plasma used for transfusion or fractionation be as free as possible of cellular blood species, such as leukocytes, red blood cells, platelets. For example, European Council Guidelines dictate that fresh frozen plasma should contain less than 6.0 x 10⁹ residual red blood cells per liter, less than 0.1 x 10⁹ residual leukocytes per liter, and less than 50 x 10⁹ residual platelets per liter. There is therefore a growing demand for blood processing and storage systems that can treat plasma in a way that removes virtually all cellular blood species.

EP-A-0976913 discloses a set of bags functioning in a closed circuit with a primary bag 2 for receiving blood and connected to a first receiving bag 17 via a leukocyte filter 11. Second and third secondary bags 22 and 24 are connected to the bag 17 and, via line 19, to the primary bag 2.

### Summary of the Invention

The invention provides systems and methods for harvesting plasma that is free or virtually free of cellular blood species.

The invention provides blood processing systems and methods that include a first container to receive blood for centrifugal processing into a first component and a second component comprising plasma. The systems and methods also include a second container to receive the second component from the first container. The systems and methods further include a filter to remove cellular species from the second component.

In one embodiment, the systems and methods include a filter to remove leukocytes from blood in an upstream flow direction from the first container. The blood may, e.g., comprise whole blood.

In one embodiment, the systems and methods also include a filter to remove leukocytes from the first component in a downstream flow direction from the first container. The first component may include, e.g., red blood cells. A transfer container to receive the first component after filtration may be provided.

In one embodiment, the filter to remove cellular species from the second component is located in an upstream flow direction from the second container, e.g., between the first container and the second container.

In one embodiment, the filter to remove cellular species from the second component is located in a downstream flow direction from the second container, e.g., between the second container and a downstream transfer container, which receives the second component after passage through the filter.

In one embodiment, the systems and methods include an auxiliary container that holds an additive solution. In one arrangement, the auxiliary container communicates with the first container, e.g., for mixing the additive solution with the first component.

In another arrangement, the auxiliary container communicates with both the first and second containers. In this arrangement, the filter to remove cellular species from the second component may be located between the second container and the auxiliary container. In this arrangement, the auxiliary container can hold an additive solution, e.g. for mixing with the first component and, upon emptying, can also serve as a transfer container to receive the second component after passage through the filter.

Other features and advantages of the invention will be pointed out in, or will be apparent from, the drawings, specification and claims that follow.

### Description of the Drawings

Figs. 1 to 7 are alternative forms of a first category of a blood processing and storage system that includes a finishing filter to collect a plasma component that is free or virtually free of cellular blood species, such as red blood cells, platelets, and leukocytes, the system also including a leukocyte reduction filter to collect red blood cells that have a reduced population of leukocytes;
Figs. 9 and 10 are alternative forms of a second category of a blood processing and storage system that includes a finishing filter to collect a plasma component that is free or virtually free of cellular blood species, such as red blood cells, platelets, and leukocytes, the system also including a leukocyte reduction filter to collect red blood cells that have a reduced population of leukocytes, the system also collecting a platelet concentrate;
Figs. 11 to 13 are alternative forms of a third category of a blood processing and storage system that includes a finishing filter to collect a plasma component that is free or virtually free of cellular blood species, such as red blood cells, platelets, and leukocytes, the system also including a leukocyte reduction filter to collect red blood cells that have a reduced population of leukocytes, the system also collecting a buffy coat rich in platelets;
Fig. 14 is an exploded perspective view of the leukocyte reduction filter that forms a part of the systems shown, e.g. in Figs. 7 to 10, 12, and 13, showing inlet and outlet ports that pass through a unitary peripheral seal;
Fig. 15 is an assembled perspective view of the leukocyte reduction filter shown in Fig. 14;
Fig. 16 is an assembled perspective view of an alternative embodiment of an leukocyte reduction filter that can form a part of the systems shown, e.g. in Figs. 7 to 10, 12, and 13, showing inlet and outlet ports that do not pass through the unitary peripheral seal;
Fig. 17 is an exploded perspective view of the finishing filter that can form a part of the systems shown, e.g. in Figs. 1 to 13, that, in use removes blood cell species from plasma prior to storage;
Fig. 18 is an assembled top plane view of the finishing filter shown in Fig. 17; and
Fig. 19 is an assembled side view of the finishing filter shown in Fig. 17.

The invention is not limited to the details of the construction and the arrangements of parts set forth in the following description or shown in the drawings. The invention can be practiced in other embodiments and in various other ways. The terminology and phrases are used for description and should not be regarded as limiting.

### Description of the Preferred Embodiments

### I. Systems and Methods for Collecting Cell-Free Plasma

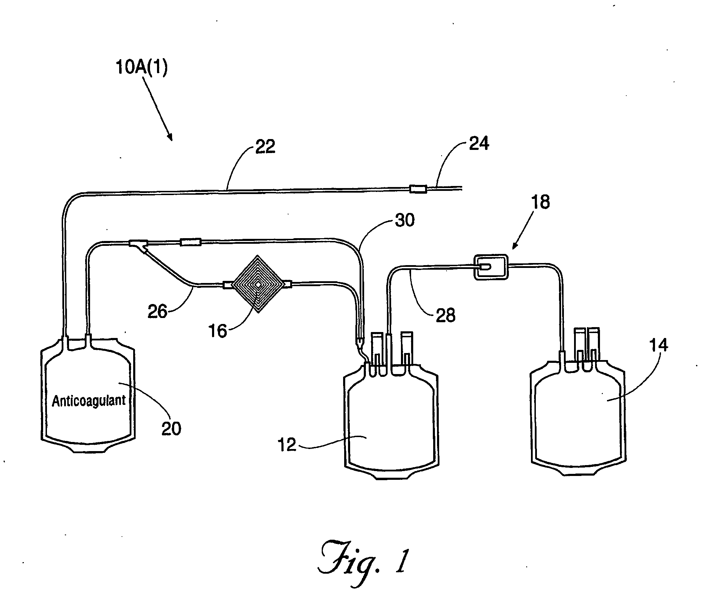

The Figs. 1 to 13 show various categories of blood collection and storage systems 10 that embody features of the invention.

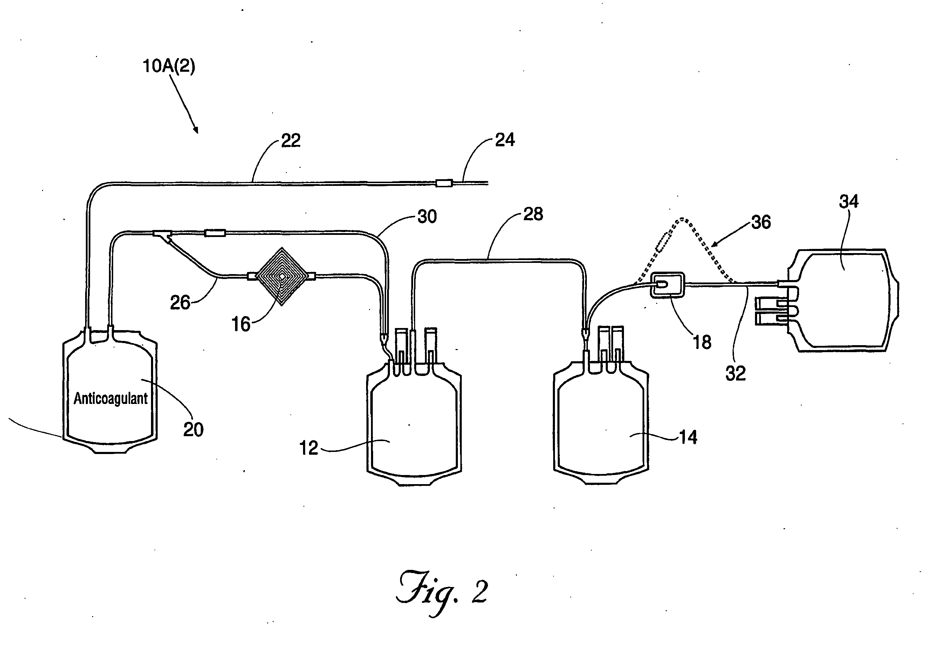

Each system 10 (see, e.g., Fig. 1) includes some form of a blood processing container 12. In use, the blood processing container 12 receives a unit of whole blood for centrifugal separation. Each system 10 also includes some form of at least one transfer container 14, which is attached to the blood processing container 12 by flexible transfer tubing 28. In use, the transfer container 14 receives a targeted blood component separated during centrifugation in the blood processing container 12. While not shown, it is to be understood that the system 10 shown in Fig.1, as well as the other Figs. 2 to 13, includes conventional external clamps and in-line frangible cannulas, which are manipulated in conventional fashion to control fluid flow within the given system 10, as is well understood by persons of skill in the art of blood processing.

The containers 12 and 14 and transfer tubing associated with each system can all be made from conventional approved, flexible, medical grade plastic materials, such as polyvinyl chloride plasticized with di-2-ethylhexyl-phthalate (PVC-DEHP). The containers 12 and 14 are formed using conventional heat sealing technologies, e.g., radio frequency (RF) heat sealing. Each system constitutes a sterile, "closed" system, as judged by the applicable standards. Each system is intended to be a disposable, single use item.

The systems 10 share at least one common objective: that is, to process a unit of whole blood in the processing container 12 to obtain a plasma component for transfer to the transfer container 14. The plasma component is characterized in that (i) it is suited for long term storage and transfusion (either in the transfer container 14 or in another separate storage container, as will be described); and (ii) it is free or virtually free of cellular blood species, such as red blood cells, platelets, and leukocytes. This plasma component obtained by the systems 10 will, in shorthand, be called "cell-free plasma."

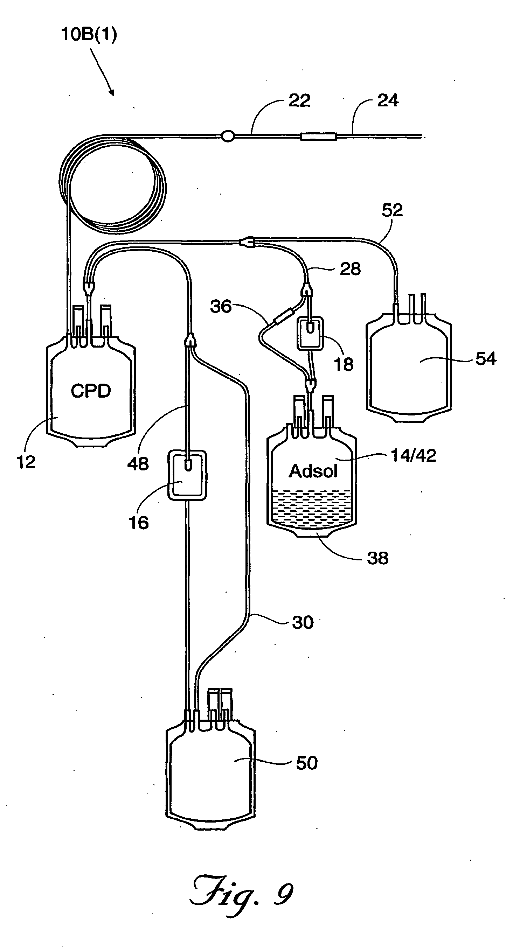

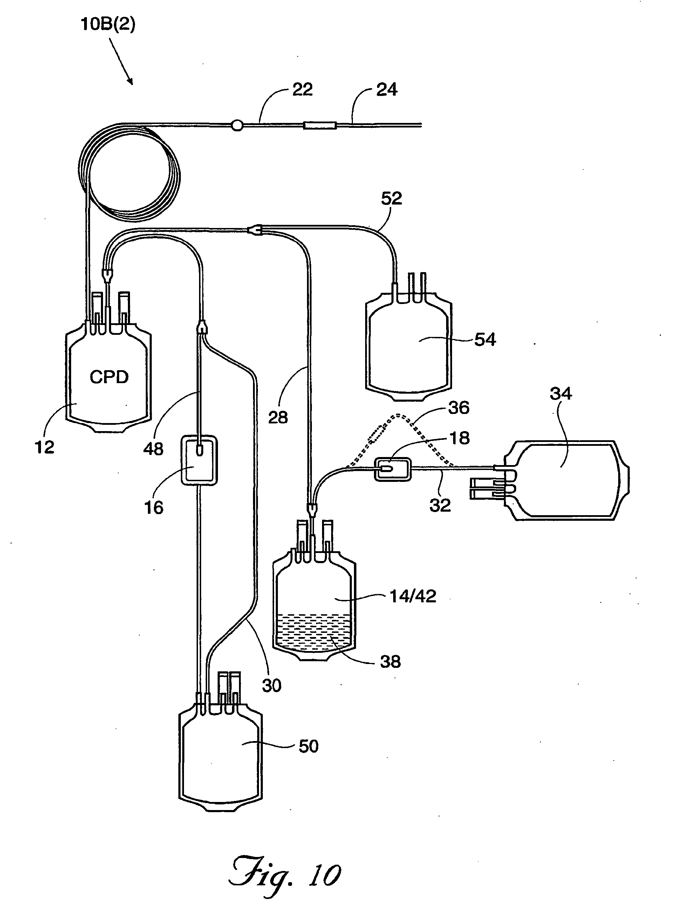

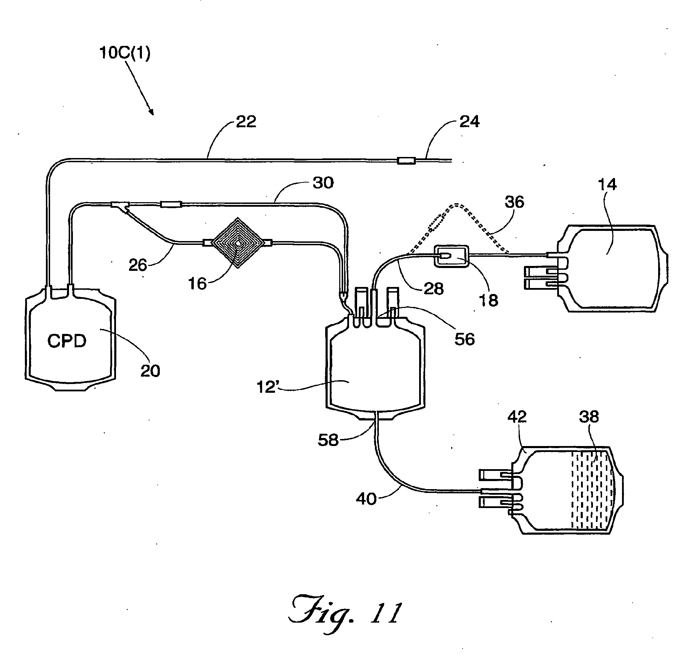

The systems 10 can be configured to harvest other desired blood components, as well. In this respect, the systems 10 fall into three general categories 10A, 10B, and 10C. The first category 10A (exemplified in various forms in Figs. 1 to 8) collects red blood cells, as well as cell-free plasma. The second category 10B (exemplified in various forms in Figs. 9 and 10) collects red blood cells and a platelet concentrate as well as cell-free plasma. The third category 10C (exemplified in various forms in Figs. 11 to 13) collects red blood cells and a buffy coat rich in platelets, as well as cell-free plasma.

Exemplary embodiments of each system category and the associated methods of using them will now be described.

### A. Category 1: Collecting Cell-Free Plasma and Red Blood Cells

The systems 10A in this category (see Figs. 1 to 8) obtain red blood cells and cell-free plasma.

Desirably, the red blood cells obtained are themselves free or virtually free of leukocytes, or have otherwise had the population of leukocytes reduced, a condition that will be called "leuko-reduced." The systems 10A achieve this result either by removing leukocytes from the whole blood before undergoing centrifugal separation in the blood processing container 12 or by removing leukocytes from the red blood cells after undergoing centrifugal separation in the blood processing container 12. In the illustrated embodiments, the leukocytes are removed by adsorption using a leukocyte-reduction filter 16 containing a fibrous filtration medium, as will be described in greater detail later.

In the illustrated embodiment, the cell-free plasma is obtained by exclusion using a finishing filter 18 that contains a membrane filtration medium, as will also be described in greater detail later.

### 1. Leukocyte Reduction of Whole Blood

Fig. 1 shows a system 10A(1) that collects leukocyte-reduced red blood cells and cell-free plasma. In this arrangement, the leukocyte population of the whole blood is reduced before centrifugal separation is achieved. Due to this, the system 10A(1) includes a blood collection container 20 separate from the blood processing container 12. The blood collection container 20 carries a suitable anticoagulant, e.g., CPD. Donor tubing 22, carrying a phlebotomy needle 24, is integrally attached to the whole blood collection container 20.

The blood collection container 20 is coupled by transfer tubing 26 to the blood processing container 12. The transfer tubing 26 carries an in-line leukocyte-reduction filter 16.

The transfer tubing 28 integrally couples the transfer container 14 for collecting cell-free plasma to the blood processing container 12. The transfer tubing 28 carries an in-line finishing filter 18.

In manipulating the system 10A(1), whole blood is collected through the donor tubing 22 in the blood collection container 20. The anticoagulant mixes with the collected whole blood. After whole blood collection, the donor is disconnected. The donor tubing 22 is sealed and severed, and the anticoagulated whole blood is drained by gravity through the transfer tubing 26 into the blood processing container 12. The in-line leukocyte-reduction filter 16 reduces the population of leukocytes in the whole blood during its transit to the blood processing container 12.

Following filtration, residual air can be vented from the blood processing container 12 through branch tubing 30, bypassing the filter 16, and into the blood collection container 20. A whole blood sample can also be collected in the branch tubing 30, as is disclosed in copending United States Patent Application Serial No. 09/0888,231, filed June 1, 1998, and entitled "Blood Collection Systems and Methods Employing an Air Venting Blood Sample Tube," which is incorporated herein by reference. The transfer tubing 26 and branch tubing 30 and branch tubing are then sealed and severed, to separate the blood collection container 20 from the blood processing container 12.

The blood processing container 12, together with the still integrally attached downstream transfer container 14, finishing filter 18, and tubing 28, are placed into a conventional blood centrifuge. In the centrifuge, the whole blood is centrifugally separated into red blood cells and blood cell-poor plasma. Since the system is intended to harvest plasma that is virtually free of blood cells, the rate of rotation is selected (employing a so-called "hard spin") to separate a majority of the platelets out of the plasma, along with the red blood cells. As a result, a majority of the platelets reside with the red blood cells, providing blood cell-poor plasma.

Following centrifugal separation, the blood cell-poor plasma is expressed from the blood processing container 12 through the transfer tubing 28 into the transfer container 14. A conventional V-shaped plasma press can be used for this purpose.

While being expressed from the blood processing container 12, the finishing filter 18 removes all or virtually all residual red blood cells and platelets from the plasma (and which, due to the larger size of leukocytes, incidently will remove any residual leukocytes as well).

The transfer tubing 28 can now be sealed and severed close to the transfer container 14. In this arrangement, the transfer container 14 also serves as the storage container for the cell-free plasma.

If desired (see Fig. 2), the plasma can be conveyed by gravity flow through the finishing filter 18 after being expressed by the plasma press from the blood processing container 12. This arrangement protects the finishing filter 14 from exposure to elevated pressures occasioned by use of the plasma press. This arrangement also expedites the transfer of plasma from the blood processing container 12 to the transfer container 14.

As shown in Fig. 2, the system 10A(2) can alternatively include transfer tubing 32 coupled between the transfer container 14 and a collection container 34. In this embodiment, the transfer tubing 32 carries the in-line finishing filter 18. That is, no filtration occurs in the process of transferring plasma from the blood processing container 12 through the transfer tubing 28 into the transfer container 14.

In this arrangement, after plasma is expressed from the blood processing container 12 by the plasma press into the transfer container 14, the transfer tubing 28 between the transfer container 14 and blood processing container 12 is severed. The transfer container 14 can then be hung upside down, to convey the plasma by gravity flow through the finishing filter 18 into the collection container 34.

Following filtration, residual air can be vented from the collection container 34 through branch tubing 36, bypassing the finishing filter 18, and into the transfer container 14. In this arrangement, the collection container 34 serves as the storage container for the cell-free plasma.

If desired, either system shown in Figs. 1 and 2 can be further modified to include an additive solution 38 for the red blood cells. One such solution is disclosed in Grode et al U.S. Patent 4,267,269, which is sold by Baxter Healthcare Corporation under the brand name ADSOL^{®} Solution. Other examples include SAGM solution or CPDA-1 solution.

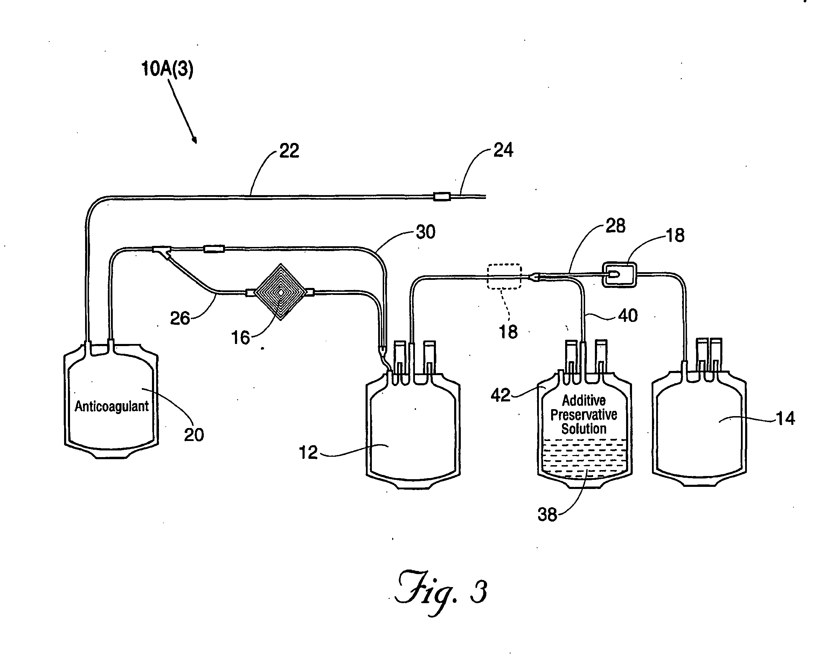

As Fig. 3 shows, the system 10A(1) in Fig. 1 can be modified to form system 10A(3) to include a transfer tubing branch 40 joining the transfer tubing 28 and itself integrally coupled to an auxiliary container 42. The auxiliary container 42 carries the additive solution 38 for red blood cells. After transfer of the plasma from the blood processing container 12 into the transfer container 14, the red blood cell additive solution 38 can be transferred from the auxiliary container 42 and mixed with the red blood cells (and platelets) remaining in the blood processing container 12. The branch transfer tubing 40 can then be sealed and severed close to the blood processing container 12. The red blood cells can be stored in the presence of the additive solution 38 in conventional fashion in the blood processing container 12.

As shown in Fig. 3, the finishing filter 18 can be located in transfer tubing 28 in a downstream flow direction from the junction with the transfer tubing 40 or, alternatively (as shown by phantom lines in Fig. 3), in an upstream flow direction from the junction.

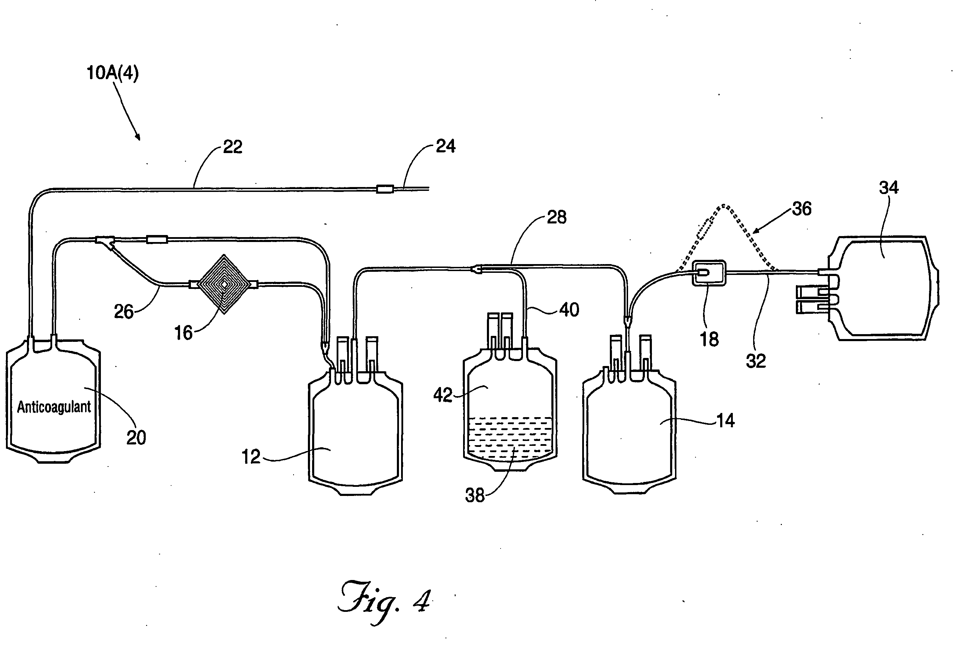

As Fig. 4 shows, the system 10A(2) shown in Fig. 2 can be modified to form a system 10A(4) that also includes a branch transfer tubing 40 and auxiliary container 42 carrying a red blood cell additive solution 38. The additive solution 38 is conveyed into the blood processing container 12 for mixing with the red blood cells (and platelets) after plasma is conveyed into the transfer container 14.

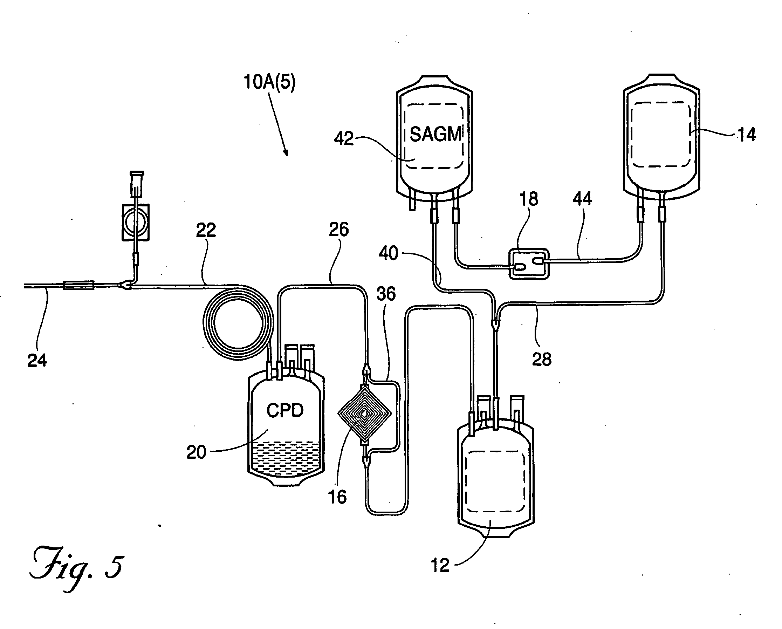

Fig. 5 shows an alternative system 10A(5) that reduces the number of containers and simplifies handling, while achieving the same results as the system 10A(4) shown in Fig. 4. In Fig. 5, the transfer tubing leg 28 couples the transfer container 14 to the blood processing container 12. The other transfer tubing leg 40 couples the auxiliary container 42 (containing the additive solution 38) to the blood processing container 12. Linking tubing 44 further couples the transfer container 14 to the auxiliary container 42. The linking tubing 44 carries a finishing filter 18.

In this arrangement, plasma is expressed by a conventional plasma press from the blood processing container 12 into the transfer container 14 through the tubing leg 28. The additive solution 38 is next transferred by gravity flow from the auxiliary container 42 into the blood processing container 12 through the tubing leg 40, for mixing with the remaining red blood cells. At this point, the transfer tubing legs 28 and 40 can be sealed and severed, to separate the blood separation container 12, which, in this arrangement serves as the storage container for the red blood cells.

Plasma can be transferred by gravity flow through the linking tubing 44, through the finishing filter 18, to the auxiliary container 42. The linking tubing 44 is sealed and severed. In this arrangement, and the auxiliary container 42 serves as the storage container for the cell-free plasma.

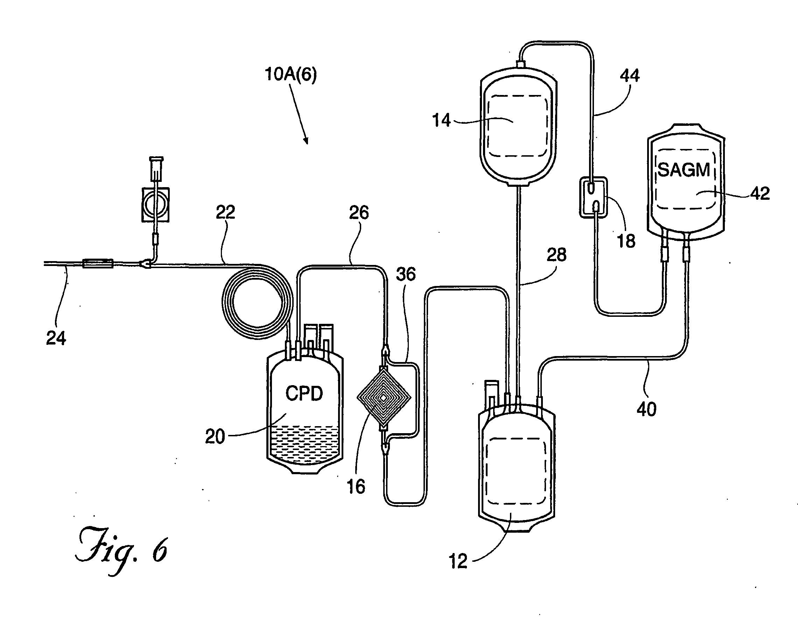

A further alternative embodiment is shown in Fig. 6. In Fig. 6, a system 10A(6) includes a transfer tubing loop 46 that communicates with the blood processing container 12. A first leg of the loop 46 serves as the transfer tubing 28, coupling the blood processing container 12 to the transfer container 14 (through a bottom seal). A second leg of the loop 46 serves as the transfer branch 40, coupling the auxiliary container 42 (containing the additive solution 38) to the blood processing container 12. A third leg of the loop serves as the linking tubing 44, coupling the transfer container 14 (through the top seal) to the auxiliary container 42. The linking tubing leg carries the finishing filter 18.

In this arrangement, plasma is expressed by a conventional plasma press from the blood processing container 12 through the first transfer leg 28 into the transfer container 14. The additive solution 38 is next transferred by gravity flow from the auxiliary container 42 into the blood processing container 12 through the second tubing leg 40, for mixing with the remaining red blood cells. At this point, the legs 28 and 40 can be sealed and severed, to separate the blood processing container 12, which, in this arrangement, serves as the storage container for the red blood cells.

Plasma can be transferred by gravity flow through the linking leg 44, through the finishing filter 18 to the auxiliary container 42. The second leg is sealed and severed. In this arrangement, as in Fig. 5, the auxiliary container 42 serves as the storage container for the cell-free plasma.

### 2. Leukocyte Reduction of Red Blood Cells

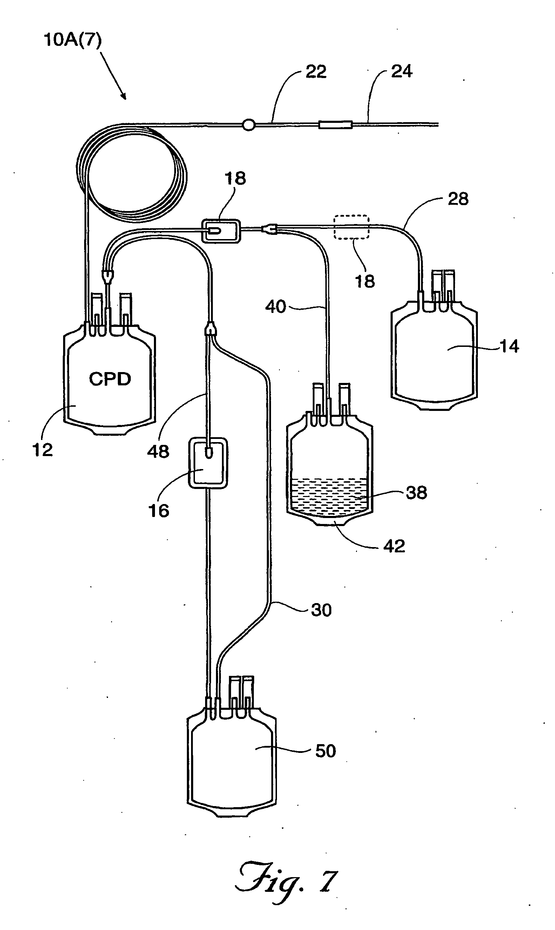

Fig. 7 shows a system 10(7) that collects leukocyte-reduced red blood cells and cell-free plasma. In this arrangement, the leukocyte population of the red blood cells is reduced after centrifugal separation of red blood cells from whole blood. Due to this, the blood processing container 12 also serves as a blood collection container. The blood processing container 12 carries a suitable anticoagulant, e.g., CPD. Donor tubing 22, carrying a phlebotomy needle 24, is also integrally attached to the whole blood processing container 12.

The transfer tubing 28 integrally couples the transfer container 14 for cell-free plasma to the blood processing container 12. The transfer tubing 28 carries an in-line finishing filter 18.

Transfer tubing 48 also integrally couples a transfer container 50 for red blood cells to the blood processing container 12. The transfer tubing 48 carries an in-line leukocyte-reduction filter 16 for removing leukocytes from red blood cells.

As Fig. 7 shows, the system 10A(7) can optionally further include the transfer tubing branch 40 joining the transfer tubing 28 and itself integrally coupled to an auxiliary container 42. The auxiliary container 42 carries an additive solution 38 for red blood cells.

As Fig. 7 shows, the finishing filter 18 can be located in transfer tubing 28 in a downstream flow direction from the junction with the transfer tubing 40 or, alternatively (as shown by phantom lines in Fig. 3), in an upstream flow direction from the junction.

In manipulating the system, whole blood is collected through the donor tubing 22 in the blood processing container 12. The anticoagulant mixes with the collected whole blood. After collection, the donor is disconnected. The donor tubing 22 is sealed and severed. A whole blood sample can also be collected in the donor tubing 22.

The blood processing container 12, together with the still integrally attached downstream containers 14 and 48 and tubing, are placed into a conventional blood centrifuge. In the centrifuge, the whole blood is centrifugally separated into red blood cells and blood cell-poor plasma. As just described, a "hard spin" is used to separate a majority of the platelets out of the plasma, along with the red blood cells. As a result, a majority of the platelets reside with the red blood cells, providing blood cell-poor plasma.

Following centrifugal separation, the blood cell-poor plasma is expressed from the blood processing container 12 through the transfer tubing 28 into the transfer container 14. As previously described, a conventional V-shaped plasma press can be used for this purpose.

While being expressed from the blood processing container 12, the finishing filter 18 removes all or virtually all residual red blood cells and platelets from the plasma (and which, due to the larger size of leukocytes, incidently will remove any residual leukocytes as well). The transfer tubing 28 can now be sealed and severed close to the transfer container 14. In this arrangement, the transfer container 14 also serves as the storage container for the cell-free plasma.

After transfer of the plasma from the blood processing container 12 into the transfer container 14, the red blood cell additive solution 38 (if present) can be transferred from the auxiliary container 42 and mixed with the red blood cells (and platelets) remaining in the blood processing container 12. The branch transfer tubing 40 can then be sealed and severed close to the blood processing container 12.

The red blood cells and additive solution 38 are then transferred from the blood processing container 12 through the transfer tubing 48 and filter 16 into the red blood cell transfer container 50. Residual air can be vented from the red blood cells collection container 50 through the branch path 30 into the blood processing container 12. Samples can also be collected in the path 30. The transfer tubing 48 can be sealed and severed close to the red blood cell collection container 50. The red blood cells can be stored in the presence of the additive solution 38 in conventional fashion in the red blood cell collection container 50.

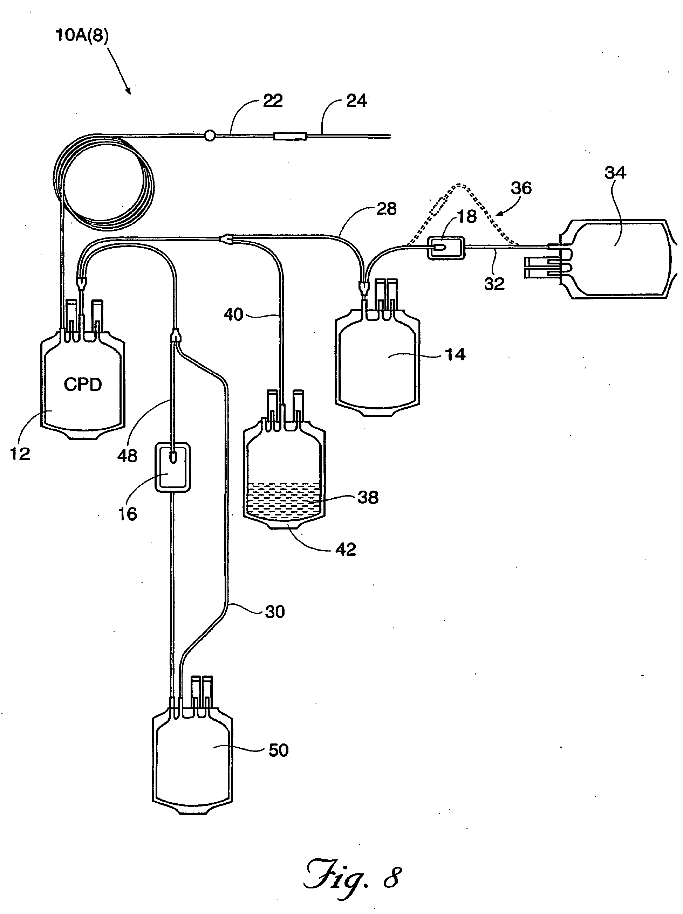

If desired, the plasma can be conveyed by gravity flow through the finishing filter 18 after being expressed from the blood processing container 12. As shown in Fig. 8, the system 10A(8) can include transfer tubing 32 coupled between the transfer container 14 and a collection container 34. The transfer tubing 32 carries the in-line finishing filter 18. In this arrangement, after plasma is expressed from the blood processing container 12 into the transfer container 14, the transfer tubing 28 between the transfer container 14 and blood processing container 12 can be severed. The transfer container 14 can then be hung upside down, to convey the plasma by gravity flow through the transfer tubing 32 and the finishing filter 18. Following filtration, residual air can be vented from the collection container through branch tubing 36, bypassing the filter 18, and into the transfer container 14. In this arrangement, the collection container 34 serves as the storage container for the cell-free plasma.

### B. Category 2: Collecting Cell-Free Plasma, Red Blood Cells, and Platelets

The systems 10 (B) in this category (see Figs. 9 and 10) obtain red blood cells, cell-free plasma, and a platelet concentrate.

As in the first category of systems 10A, the red blood cells obtained by the second category of systems 10B are themselves desirably free or virtually free of leukocytes, or are otherwise leuko-reduced. The systems 10B achieve this result by removing leukocytes from the red blood cells after undergoing centrifugal separation in the blood processing container 12, desirably by depth filtration, as will be described later.

In the illustrated embodiment, the cell-free plasma is obtained by exclusion using a finishing filter 18 that contains one or more membrane filter layers, as will be described in greater detail later.

The system 10B(1) shown in Fig. 9 is in many structural respects similar to the system shown in Fig. 7. The system 10B(1) includes the blood processing container 12, which also serves as a blood collection container 20 and carries a suitable anticoagulant, e.g., CPD. Donor tubing 22, carrying a phlebotomy needle 24, is also integrally attached to the whole blood processing container 12.

In the arrangement shown in Fig. 9, the transfer container 14 that ultimately receives cell-free plasma for storage also serves as the auxiliary container 42 for holding the red blood cell additive solution 38. The transfer tubing 28 that couples the transfer container 14 to the blood processing container 12 carries an in-line finishing filter 18. An optional branch path 36 bypasses the finishing filter 18. A transfer tubing branch 52 joins the transfer tubing 28 and itself integrally coupled to another transfer container 54.

Transfer tubing 48 also integrally couples a transfer container 50 for red blood cells to the blood processing container 12. The transfer tubing 48 carries an in-line leukocyte-reduction filter 16 for removing leukocytes from red blood cells.

In manipulating the system 10B(1), whole blood is collected through the donor tubing 22 in the blood processing container 12. The anticoagulant mixes with the collected whole blood. After collection, the donor is disconnected. The donor tubing 22 is sealed and severed. A whole blood sample can also be collected in the donor tubing 22.

The blood processing container 12, together with the still integrally attached downstream containers 14, 50, and 54 and tubing, are placed into a conventional blood centrifuge. In the centrifuge, the whole blood is centrifugally separated into red blood cells and plasma rich in platelets (employing a so-called "soft spin") to retain a majority of the platelets in the plasma, outside of the red blood cells. As a result, a majority of the platelets reside with the plasma, providing platelet-rich plasma.

Following centrifugal separation, the platelet rich plasma is expressed from the blood processing container 12 through the transfer tubing 52 into the transfer container 54. A conventional V-shaped plasma press can be used for this purpose.

After transfer of the platelet-rich plasma from the blood processing container 12 into the transfer container 54, the red blood cell additive solution 38 can be transferred from the transfer container 14 and mixed with the red blood cells remaining in the blood processing container 12. The additive solution 38 can be passed through the in-line filter 18 (in a back-flushing direction) or through the path 36 bypassing the filter 18. The red blood cells and additive solution 38 are then transferred from the blood processing container 12 through the transfer tubing 48 and filter 16 into the red blood cell transfer container 50. Residual air can be vented from the red blood cells collection container 50 through the branch path 30 into the blood processing container 12. Samples can also be collected in the branch path 30. The transfer tubing 48 can be sealed and severed close to the red blood cell collection container 50. The red blood cells can be stored in the presence of the additive solution 38 in conventional fashion in the red blood cell collection container.

The transfer tubing 28 can be severed near the junction of the transfer tubing and transfer tubing branch. The remaining transfer containers 14 and 54 are returned to the centrifuge. In the centrifuge, the platelet-rich plasma is centrifugally separated in the container 54 into a concentration of platelets and platelet-poor plasma. Following centrifugation, the platelet poor plasma is expressed from the container 54 into the transfer container 14, which is now empty of the additive solution 38. A conventional v-shaped plasma press can be used for this purpose. While being expressed from the second transfer container 14, the finishing filter 18 removes all or virtually all residual red blood cells and platelets from the plasma (and which, due to the larger size of leukocytes, incidently will remove any residual leukocytes as well). The transfer tubing 28 can now be sealed and severed close to the transfer container 14. In this arrangement, the transfer container 14 (i.e., also serving as the auxiliary container 42) also serves as the storage container for the cell-free plasma.

In this arrangement, the transfer container 54 serves as the storage container for the platelets. Accordingly, it can be made of polyolefin material (as disclosed in Gajewski et al U.S. Patent 4,140,162) or a polyvinyl chloride material plasticized with tri-2-ethylhexyl trimellitate (TEHTM). These materials, when compared to DEHP-plasticized polyvinyl chloride materials, have greater gas permeability that is beneficial for platelet storage.

If desired, the plasma can be conveyed by gravity flow through the finishing filter 18 after being expressed from the blood processing container 12. As shown in Fig. 10, a system 10B(2) can include transfer tubing 32 coupled between the transfer container 14 (originally serving as the auxiliary container 42 to hold a red blood cell additive solution 38) and a collection container 34. The transfer tubing 32 carries the in-line finishing filter 18. In this arrangement, after platelet-poor plasma is expressed from the transfer container 54 into the container 14 (by now empty of the additive solution 38, as previously described), the transfer tubing 28 can be severed close to the container 14. The container 14 can then be hung upside down, to convey the plasma by gravity flow through the finishing filter 18 into the collection container 34. Following filtration, residual air can be vented from the collection container 34 through branch tubing 36, bypassing the filter 18, and into the transfer container 14. In this arrangement, the collection container 34 ultimately serves as the storage container for the cell-free plasma.

### C. Category 3: Collecting Cell-Free Plasma, Red Blood Cells, and Buffy Coat Platelets

The systems 10C in this category (see Figs. 11 to 13) harvest red blood cells, cell-free plasma, and a buffy coat rich in platelets.

As in the first and second categories of systems 10A and 10B, the red blood cells obtained by the third category of systems 10C desirably are themselves free or virtually free of leukocytes, or are otherwise leuko-reduced. The systems 10C achieve this result by using a specially designed blood separation container 12' (see Fig. 11) having both top and bottom outlets 56 and 58, and by further removing leukocytes by adsorption either from whole blood before centrifugal separation in the blood processing container 12' or from the red blood cells after undergoing centrifugal separation in the blood processing container 12'. In the illustrated embodiment, the leukocytes may be removed using an appropriate filtration medium. In this arrangement, the filtration medium desirably allows a substantial number of platelets to pass.

In the illustrated embodiment, the cell-free plasma is obtained by exclusion using a finishing filter 18 that contains one or more membrane filter layers, as will be described in greater detail later.

### 1. Leukocyte Removal From Whole Blood

Fig. 11 shows a system 10C(1) that collects leukocyte-reduced red blood cells, cell-free plasma, and a buffy coat rich in platelets. In this arrangement, the leukocyte population of the whole blood is reduced before centrifugal separation occurs. The system 10C(1) (like previously described system 10A(1)) therefore includes a blood collection container 20 separate from the blood processing container 12'. The blood collection container 20 carries a suitable anticoagulant, e.g., CPD. Donor tubing 22, carrying a phlebotomy needle 24, is integrally attached to the whole blood collection container 20.

The blood collection container 20 is coupled by transfer tubing 26 to the blood processing container 12. The transfer tubing carries an in-line leukocyte-reduction filter 16.

Transfer tubing 28 integrally couples the top outlet 56 of the blood processing container 12' to the transfer container 14 for cell-free plasma. The transfer tubing 28 carries an in-line finishing filter 18. An optional bypass branch 30 may also be provided for air venting and sampling, as has already been described.

Transfer tubing 40 integrally couples the bottom outlet 58 of the blood processing container 12' to an auxiliary container 42 holding an additive solution 38 for red blood cells.

In manipulating the system 10C(1), whole blood is collected through the donor tubing 22 in the blood collection container 20. The anticoagulant mixes with the collected whole blood. After collection, the donor is disconnected. The donor tubing 22 is sealed and severed, and the anticoagulated whole blood is expressed through the transfer tubing 26 into the blood processing container 12'. The filter 16 removes leukocytes from whole blood during its transit to the blood processing container 12'.

Following filtration, residual air can be vented from the blood processing container 12' through branch tubing 30, bypassing the filter 16, and into the blood collection container 20. A whole blood sample can also be collected in the branch tubing 30. The transfer tubing 26 and branch tubing 30 are then sealed and severed.

The blood processing container 12', together with the still integrally attached downstream containers 14 and 42 and tubing, are placed into a conventional blood centrifuge. The forces of centrifugation are controlled to separate the whole blood into a top layer of blood cell-poor plasma, a bottom layer of red blood cells, and an intermediate layer (called the buffy coat) in which mostly leukocytes and platelets reside.

Following separation in this manner, the whole blood processing container 12' is squeezed between two generally parallel plates of a plasma extractor, which is commercially available under the tradename Opti-Press^{®} System from Baxter Healthcare Corporation. The blood cell-poor plasma is expressed through the top port 56, through the finishing filter 18, into the plasma collection container 14. The red blood cells are expressed from the bottom port 58 into the container 42, where the red blood cells mix with the additive solution 38.

The location of the intermediate buffy coat layer is optically monitored, to retain the interface layer within the whole blood processing container 12'. In this way, the leukocyte and platelet population of the red blood cells and plasma can be reduced. Also, the intermediate buffy coat layer can itself be later harvested for platelets after rinsing with a platelet additive solution followed by soft centrifugation.

Following transfer of blood cell-free plasma and red blood cells from the whole blood processing container 12', air in the transfer container 14 may be vented through the bypass branch 36 into the blood processing container 12'. The top and bottom transfer tubings 28 and 40 are sealed and severed from the whole blood processing container 12'. The filtered plasma, now virtually free of cellular blood species, is stored in conventional fashion in the transfer container 14. Filtered leuokocyte-depleted red blood cells, virtually free of leukocytes or otherwise leuko-reduced, are stored in conventional fashion in the container 42, which originally served to carry the additive solution.

### 2. Leukocyte Removal From Red Blood Cells

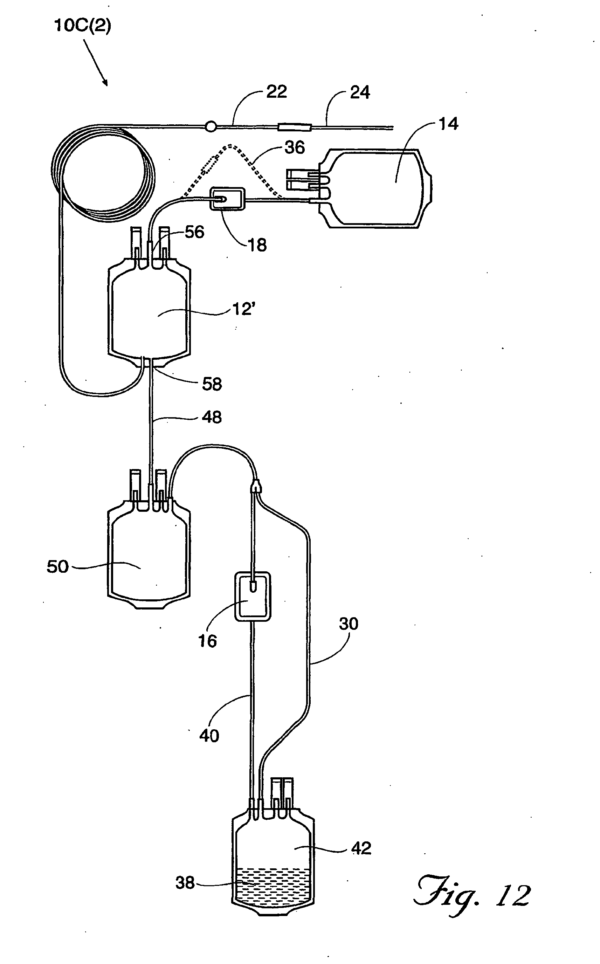

Fig. 12 shows another system 10C(2) that collects leukocyte-reduced red blood cells, cell-free plasma, and a buffy coat rich in platelets. In this arrangement, the leukocyte population of the red blood cells is reduced after centrifugal separation in the blood processing container 12'. In this arrangement, the blood processing container 12' also serves as the blood collection container 20. As such, it contains a suitable anticoagulant, e.g., CPD. Donor tubing 22, carrying a phlebotomy needle 24, is also integrally attached to the whole blood processing container 12.

In Fig. 12, the blood processing container 12' includes a top outlet 56 and a bottom outlet 58. Transfer tubing 28 integrally couples the top outlet 56 of the blood processing container 12' to the transfer container 14 for cell-free plasma. The transfer tubing 28 carries an in-line finishing filter 18. An optional bypass branch 36 may also be provided for air venting, as previously described.

Transfer tubing 48 integrally couples the bottom outlet 58 of the blood processing container 12' to transfer container 50. Further transfer tubing 40 couples the transfer container 50 to an auxiliary container 42, which holds an additive solution 38 for red blood cells. The transfer tubing 40 carries an in-line leukocyte-reduction filter 16. An optional bypass branch 30 may also be provided for air venting. Blood samples may also be collected in the path 30.

In manipulating the system shown in Fig. 12, whole blood is collected through the donor tubing 22 in the blood processing container 12'. The anticoagulant mixes with the collected whole blood. A whole blood sample can also be collected in the donor tubing 22. After collection, the donor is disconnected.

The blood processing container 12', together with the still integrally attached downstream containers 14, 42, and 50 and tubing, are placed into a conventional blood centrifuge. The forces of centrifugation are controlled to separate the whole blood into a top layer of blood cell-poor plasma, a bottom layer of red blood cells, and an intermediate layer (called the buffy coat) in which mostly leukocytes and platelets reside.

Following separation in this manner, the whole blood processing container 12' is squeezed between two generally parallel plates of a plasma extractor, which is commercially available under the tradename Opti-Press^{®} System from Baxter Healthcare Corporation. The blood cell-poor plasma is expressed through the top port 56, through the tubing 28 and finishing filter 18, into the plasma collection container 14. While being expressed from the blood processing container 12', the finishing filter 18 removes all or virtually all residual red blood cells and platelets from the plasma (and which, due to the larger size of leukocytes, incidently will remove any residual leukocytes as well).

The red blood cells are expressed from the bottom port 58 into the transfer container 50.

The location of the intermediate buffy coat layer is optically monitored, to retain the interface layer within the whole blood processing container 12'. In this way, the leukocyte and platelet population of the red blood cells and plasma can be reduced. Also, the intermediate buffy coat layer can itself be later harvested for platelets after rinsing with a platelet additive solution followed by soft centrifugation.

Following transfer of blood cell-free plasma from the whole blood processing container 12', air in the transfer container 14 may be vented through the bypass branch 36 into the blood processing container 12'. The top transfer tubing 28 is sealed and severed from the whole blood processing container 12'. The filtered plasma, now virtually free of cellular blood species, is stored in conventional fashion in the transfer container 14.

Red blood cells in the transfer container 50 are passed by gravity flow through the transfer tubing 40 and leukocyte-reduction filter 16 into the container 42. The filter 16 removes leukocytes from the red blood cells during transit to the container 42. Following filtration, residual air can be vented from the container 42 through branch tubing 30, bypassing the filter 16, and into the transfer container 50. The transfer tubing 40 is then sealed and severed. Filtered leukocyte-depleted red blood cells, virtually free of leukocytes or otherwise leuko-reduced, are stored in conventional fashion in the container 42, which originally served as the auxiliary container 42 to hold additive solution 38. Alternatively, the additive solution 38 can be originally contained in the transfer container 50 for mixing with the red blood cells prior to filtration.

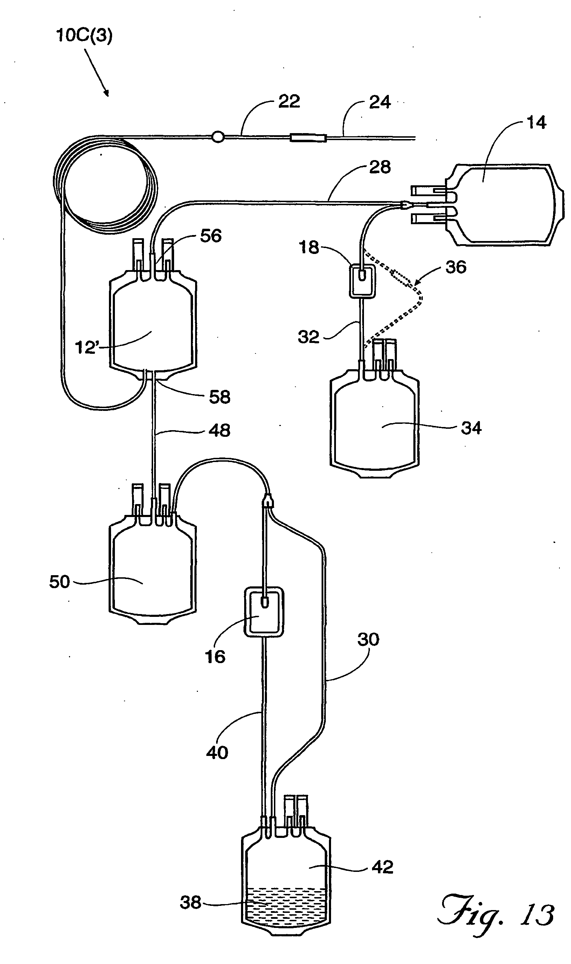

If desired, the plasma can be conveyed by gravity flow through the finishing filter 18 after being expressed from the blood processing container 12'. As shown in Fig. 13, a system 10C(3) can include transfer tubing 32 coupled between the transfer container 14 and a collection container 34. The transfer tubing 32 carries the in-line finishing filter 18. In this arrangement, after plasma is expressed from the blood processing container 12' into the transfer container 14, the transfer tubing 28 between the transfer container 14 and blood processing container 12' can be severed. The transfer container 14 can then be hung upside down, to convey the plasma by gravity flow through the finishing filter 18. Following filtration, residual air can be vented from the collection container 34 through branch tubing 36, bypassing the filter 18, and into the transfer container 14. In this arrangement, the collection container 34 ultimately serves as the storage container for the cell-free plasma. Alternatively, the additive solution 38 can be originally contained in the transfer container 50 for mixing with the red blood cells prior to filtration.

### II. Filters for Removing Leukocytes from Whole Blood or Red Cells

The filter 16 for reducing the population of leukocytes from while blood or red blood cells can be variously constructed.

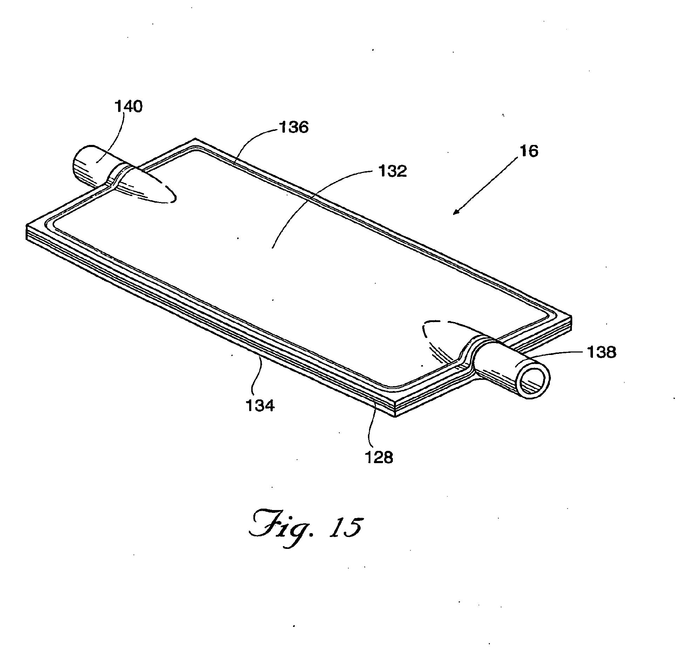

Desirably, the filter 16 includes a filtration medium contained within a flexible housing 130 (see Fig. 15) made using conventional approved medical grade plastic materials using conventional radio frequency heat sealing technology. The filter 16, being flexible, facilitates handling and reduces the incidence of damage to other components of the system during centrifugal processing. The flexible filter 16 avoids the handling and processing problems rigid filter housings have presented in the past. Unlike a rigid housing, the flexible housing 130 will not puncture associated containers, which are also made of flexible plastic materials. Unlike a rigid housing, the flexible housing 130 conforms and is compliant to stress and pressures induced during use.

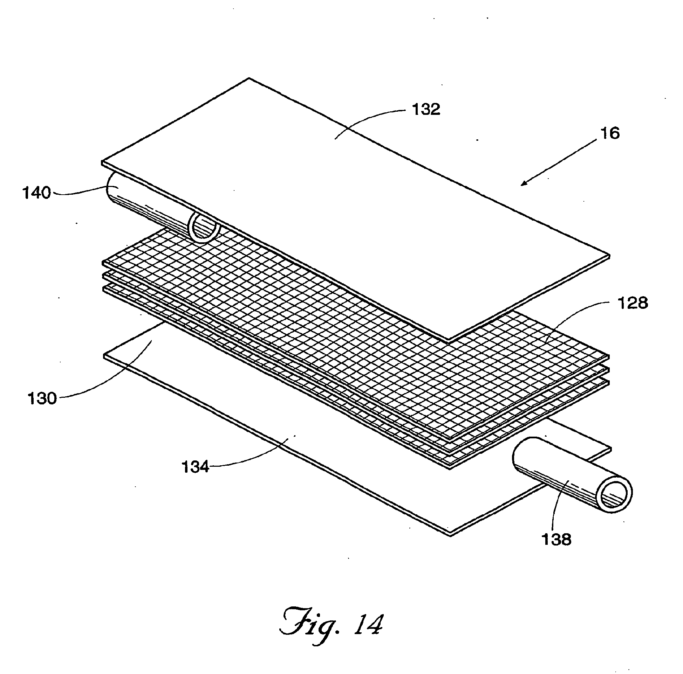

In the illustrated embodiment (see Fig. 14), the filter housing 130 comprising first and second sheets 132 and 134 of medical grade plastic material, such as polyvinyl chloride plasticized with di-2-ethylhexyl-phthalate (PVC-DEHP). Other medical grade plastic materials can be used that are not PVC and/or are DEHP-free, provided that the material heats and flows when exposed to radio frequency energy.

The filtration medium 128 is made from a fibrous material, which is sandwiched between the sheets 132 and 134. The filtration medium 128 can be arranged in a single layer or in a multiple layer stack. The medium 128 can include melt blown or spun bonded synthetic fibers (e.g., nylon or polyester or polypropylene), semi-synthetic fibers, regenerated fibers, or inorganic fibers. In use, the medium 28 removes leukocytes by depth filtration.

In the illustrated embodiment, the filtration medium 128 comprises, in the blood flow direction, a prefilter region, a main filter region, and a postfilter region. The prefilter and postfilter are made of fibrous material (e.g., polyethylene) having a pore size and fiber diameter not suited for leukocyte removal. Instead, the fibrous material of the prefilter is sized to remove gross clots and aggregations present in the blood. The fibrous material of the postfilter is sized to provide a fluid manifold effect at the outlet of the filter. In a representative embodiment, the prefilter material has a pore size of between about 15 µm to about 20 µm, and the postfilter material has a pore size of about 20 µm. The main filter region is made of a fibrous material (e.g., polyethylene) having a pore size and diameter sized to remove leukocytes by depth filtration. The material of the main filter region can have the characteristics described in Watanabe et al. United States Patent No. 4,701,267 or Nishimura et al. United States Patent No. 4,936,998, which are incorporated herein by reference.

As disclosed, the filtration medium 128 can be made symmetric, meaning that the material layers of filtration medium encountered during flow through the medium 128 are the same regardless of the direction of flow. Thus, either side of the medium 128 can serve as an inlet or an outlet. The symmetric nature of the filtration medium 128 further simplifies manufacture, as it is not necessary to differentiate between "inlet" and "outlet" side of the filtration medium 128 or "inlet" or "outlet" orientation of the sheets 132 and 134.

According to the invention, a unitary, continuous peripheral seal 136 is formed by the application of pressure and radio frequency heating in a single process to the two sheets 132 and 134 and filtration medium 128. The seal 136 joins the two sheets 132 and 134 to each other, as well as joins the filtration medium 128 to the two sheets 132 and 134. The seal 136 integrates the material of the filtration medium 128 and the material of the plastic sheets 132 and 134, for a reliable, robust, leak-proof boundary. Since the seal 136 is unitary and continuous, the possibility of blood shunting around the periphery of the filtration medium 130 is eliminated.

At its surface, along the sheets 132 and 134, the seal 136 comprises mostly the material of the sheets 132 and 134. With increasing distance from the surface, the seal 136 comprises a commingled melted matrix of the material of the sheets and the material of the filtration medium. This is believed to occur because the sheet material, which is electrically heated and caused to flow by the applied radio frequency energy, is further caused by the applied pressure to flow into and penetrate the interstices of the medium. The heated sheet material that flows under pressure into the interstices of the medium causes the medium itself to melt about it.

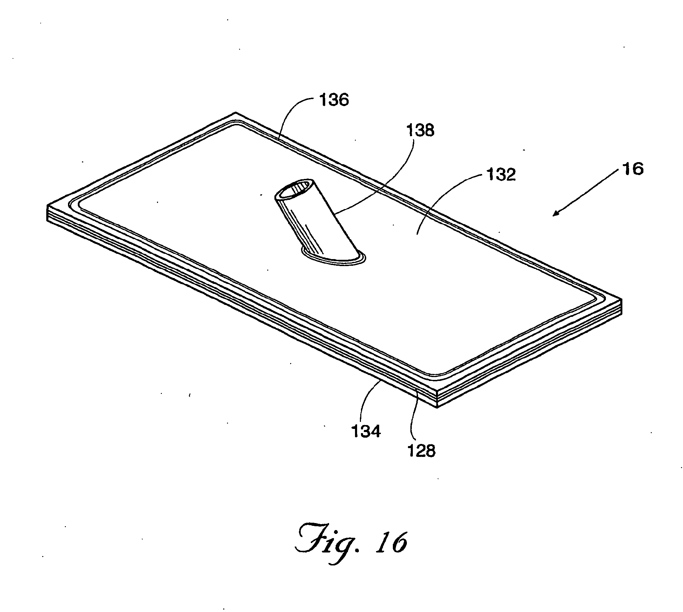

The filter 120 also includes inlet and outlet ports 138 and 140. The ports 138 and 140 comprise tubes made of medical grade plastic material, like PVC-DEHP. As Fig. 15 shows, the ports 138 and 140 can be located in the integrated peripheral seal 136, and be sealed in place at the same time that the unitary peripheral seal 136 is formed. Alternatively (see Fig. 16), the ports 138 and 140 can be inserted and sealed to each sheet 132 and 134 in a separate assembly process before the unitary peripheral seal is formed, in the manner shown in Fischer et al. U.S. Patent 5,507,904. Still alternatively, the ports 138 and 140 can comprise separately molded parts that are heat sealed by radio frequency energy over a hole formed in the sheets.

The symmetric orientation of filtration medium 128, described above, makes the filter 16 "non-directional." The port can be oriented to serve either as an inlet port or an outlet port, with the other port serving, respectively, as the corresponding outlet port or inlet port, and vice versa.

Further details of the filter 16 can be found in copending United States Patent Application Serial No. 09/593,782, filed June 14, 2000 and entitled "Blood Collection Systems Including an Integral Filter," which is incorporated herein by reference.

The filter housing 130 could, alternatively, comprise a rigid medical grade plastic material (e.g., as Figs. 1 to 6 show). However, use of flexible materials for the housing better protects the tubing and containers in contact with the housing, from damage, particular when undergoing centrifugation.

### III. Filters for Removing Cellular Blood Species from Plasma

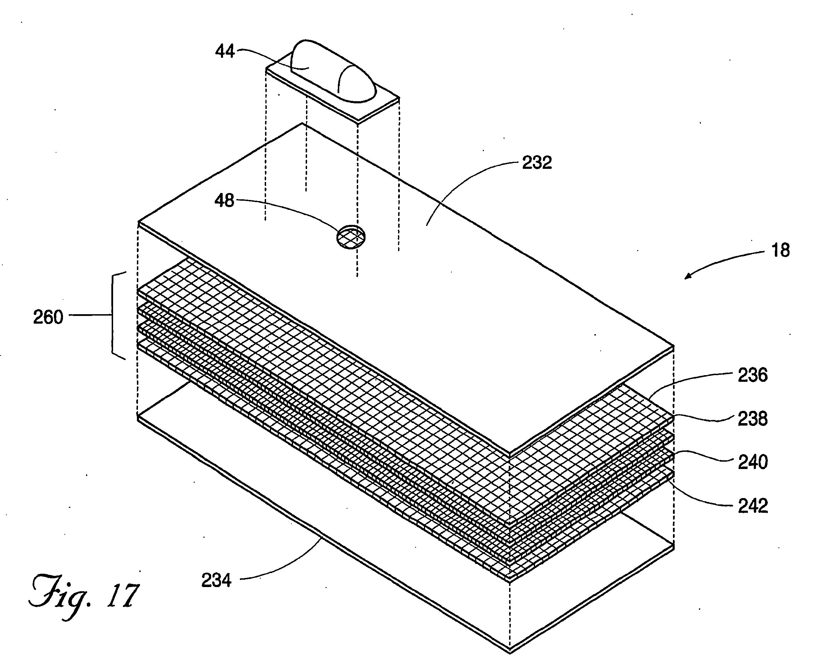

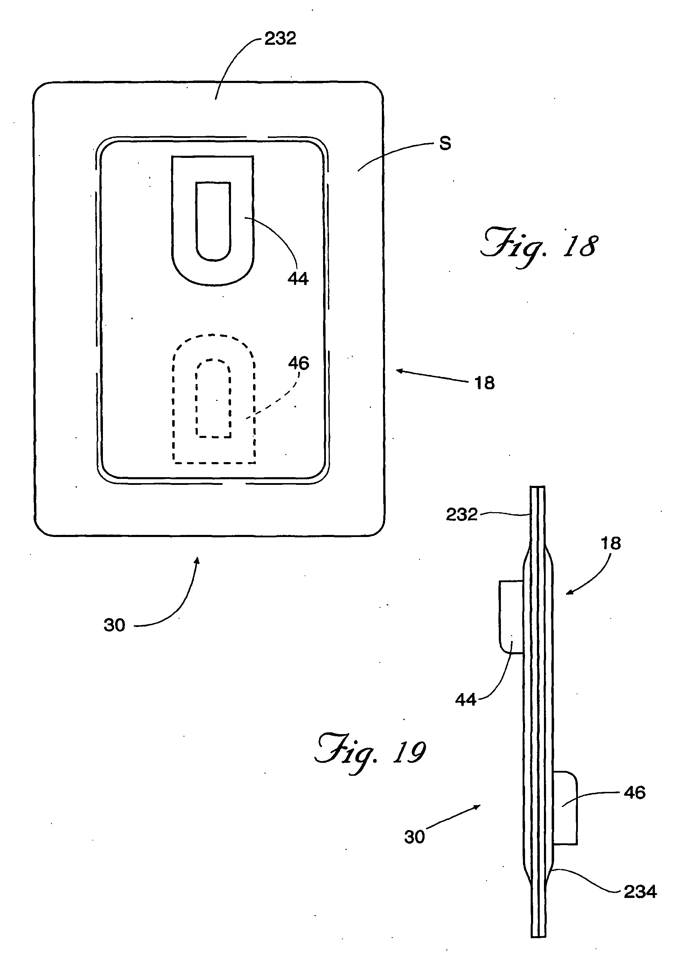

The finishing filter 18 (see Figs. 18 and 19) can likewise be variously constructed. Desirably, like the filter 16, the filter media 260 of the finishing filter 18 is also enclosed within a filter housing 230 (see Fig. 17) comprising first and second sheets 232 and 234 of flexible, medical grade plastic material, such as polyvinyl chloride plasticized with di-2-ethylhexyl-phthalate (PVC-DEHP). A peripheral seal S (see Fig. 18), formed using conventional radio frequency heat sealing technology, joins the sheets 232 and 234 about the filter media 260. Other medical grade plastic materials can be used that are not PVC and/or are DEHP-free, provided that the material heats and flows when exposed to radio frequency energy.

The pore size of the filter media 260 of the finishing filter 18 is tailored to remove by exclusion the red blood cell and platelet species of blood cells typically found in plasma.

The composition of the media 260 can vary. For examples, hydrophilic membranes made from nylon, acrylic copolymers, polysulfone, polyvinylidene fluoride, mixed cellulose esters, and cellulose ester can be used to remove red blood cells and platelets by exclusion. Non-hydrophilic membranes can also be treated to serve as a membrane for the filter media. Material selection takes into account customer preferences, performance objectives, and manufacturing requirements, including sterilization techniques.

In the illustrated and preferred embodiment, (see Fig. 17), four layers 236, 238, 240, and 242 make up the filter media 260. The four layers 236, 238, 240, and 242 are arranged, one on top of the other, in the order of blood flow through the filter 18.

The first layer 236 comprises a prefilter material. The prefilter layer 236 serves to remove fibrin clots and other large size aggregates from the plasma, but may also retain cellular blood species by affinity. The composition of the prefilter layer 36 can vary and can comprise, e.g., fibers of glass or polyester. In the illustrated embodiment, the prefilter layer 236 comprises a borosilicate microfiber glass material with an acrylic binder resin. This material is commercially available from Millipore, under the product designation AP15 or AP20. The AP15 material is preferred, as it is less porous than the AP20 material and has been observed to provide better flow rates than AP20 material. For best flow rate results, the glass fiber prefilter layer 236 should be oriented with the gross surface facing in the upstream flow direction and the fine surface facing in the downstream flow direction.

The second and third filter media layers 238 and 240 preferably possess pore sizes which are approximately ten-fold smaller than the size of leukocytes, and which decrease in the direction of flow. Due to their pore size, the second and third filter media layers 238 and 240 remove red blood cells and platelets (and incidently also leukocytes) by exclusion. In the illustrated embodiment, the second and third layers 238 and 240 comprise hydrophilic polyvinylidene fluoride (PVDF) membranes.

In a preferred embodiment, the PVDF material of the second filter media layer 238 has an average pore size of about 1.0 µm and a porosity sufficient to sustain an adequate flow of plasma through the filter 20, without plugging, which can be characterized by a bubble point (derived using water) in a range between about 8.5 psi and about 13 psi. This PVDF material is commercially available from Millipore under the trade designation CVPPB hydrophilic DURAPORE™ Membrane.

In the preferred embodiment, the PVDF material of the third filter media layer 240 has a smaller average pore size of about 0.65 µm. The layer 40 also has a porosity sufficient to sustain an adequate flow of plasma through the filter 18, without plugging, which can be characterized by a bubble point (derived using water) in a range of about 15.5 to about 20.6 psi. This PVDF material is commercially available from Millipore under the trade designation DVPP hydrophilic DURAPORE™ Membrane.

The bottommost, fourth layer 242 comprises a mesh material made, e.g., from a polyester or polypropylene material. The mesh material of the fourth layer 242 provides mechanical support for the filter. The mesh material of the fourth layer 242 also prevents the PVDF material of the third filter media layer 240 from sticking, during use, to the PVC sheet 234 along the outlet of the filter. Alternatively, the fourth layer 242 could be substituted by a roughened finished surface on the internal side of the downstream sheet 234 of the housing 230.

The finishing filter 18 includes inlet and outlet ports 244 and 246. In the illustrated embodiment (see Figs. 17, 18, and 19), the ports 244 and 246 comprise separately molded parts that are heat sealed by radio frequency energy over a hole 248 formed in the sheets 232 and 234, preferably before the peripheral seal S is created. Alternatively, the ports 244 and 246 can comprise tubes made of medical grade plastic material, like PVC-DEHP. In this arrangement, the tubes are inserted and sealed to each sheet 232 and 234 in a separate assembly process before the peripheral seal S is formed, in the manner shown in Fischer et al. U.S. Patent 5,507,904, which is incorporated herein by reference.

In use, the inlet port 244 conveys plasma into contact with the prefilter layer 236. The axis of the inlet port 244 is generally parallel to the plane of the layer 236.

The plasma flows through the prefilter layer 236 and through the second and third PVDF layers 238 and 240. There, removal of red blood cells and platelets (and, incidently, leukocytes) occurs by exclusion. The outlet port 246 conveys virtually blood cell free plasma from the second and third PVDF filter layers 238 and 240, through the mesh material 242.

Further details of the finishing filter 18 can be found in copending United States Patent Application Serial No. 09/540,935, filed March 31, 2000, and entitled "Systems and Methods for Collecting Plasma that is Free of Cellular Blood Species," which is incorporated herein by reference.

The filter housing 230 could, alternatively, comprise a rigid medical grade plastic material. However, use of flexible materials for the housing better protects the tubing and containers in contact with the housing, from damage, particular when undergoing centrifugation.

Features and advantages of the invention are set forth in the following claims.

## Claims

1. A blood processing system comprising
a first container (12') to receive blood for centrifugal processing into a first component comprising red blood cells and a second component comprising plasma,
a first filter (18) to remove one or more of leukocytes, red blood cells and platelets from the second component,
a second container (64) to receive the second component from the first container,
a third container (50) to receive the first component from the first container (12'), said third container (50) located in a downstream flow direction from said first container,
a fourth container (42) including an additive solution (38) said fourth container located in a downstream flow direction from said third container, and
a second filter (16) to remove leukocytes from the first component, wherein the second filter is located downstream of said third container.

2. The blood processing system of claim 1 further comprising a tubing connector located between said third container (50) and said second filter (16) and further comprising a bypass tubing branch (30) for bypassing fluid flow through said second filter (16) said bypass branch (30) having a first end and a second end, wherein said first end is in communication with said connector and said second end is in communication with said fourth container.

3. The blood processing system of claim 1 further including a filter to remove leukocytes from blood in an upstream flow direction from the first containe (12').

4. The blood processing system of claim 1 wherein said second filter (16) is located between said third container (50) and said fourth container (42)

5. The blood processing system of claim 1 wherein said first filter (18) is located in an upstream flow direction from the second container (14)

6. The blood processing system of claim 1 wherein the first filter (18) is located between the first container 12' and the second container (14)

7. The blood processing system of claim 1 wherein the first filter (18) is located in a downstream flow direction from the second container 14.

8. The blood processing system of claim 1 further including a transfer container (34) communicating with the second container (14) in a downstream flow direction from the second container (14).

9. The blood processing system of claim 8 wherein the first filter (18) is located between the second container (14) and the transfer container (34).

10. The blood processing system of claim 6 wherein said first filter (18) has an inlet and an outlet, said system further comprising a bypass tubing branch (36) for bypassing fluid flow through said first filter (18), said bypass branch (36) having a first end and a second end, wherein said first end is located between said first container and said filter inlet, and said second end is located between said filter outlet and said second container (14).

11. The blood processing system of claim 9 wherein said first filter (18) has an inlet and an outlet, said system further comprising a bypass tubing branch (36) for bypassing fluid flow through said first filter (18) said bypass branch (36) having a first end and a second end, wherein said first end is located between said second container and said filter inlet, and said second end is located between said filter outlet and said transfer container (34)

12. The blood processing system of claim 1 wherein said first container (12') further comprises a top outlet port (56) and a bottom outlet port (58).

13. The blood processing system of claim 12 wherein said second container (14) comprises an inlet port, and wherein said top outlet port (56) of said first container (12') is in communication with said second container inlet port.

14. The blood processing system of claim 12 wherein said third container (50) comprises an inlet port, and wherein said bottom outlet port (58) of said first container (12') is in communication with said third container inlet port

15. A blood processing method comprising processing whole blood using a system as defined in claim 1.

## Patentansprüche

1. Blutverarbeitungssystem, umfassend
einen ersten Behälter (12'), um Blut zur Fliehkraftbearbeitung in einen ersten Bestandteil, umfassend rote Blutkörperchen, und einen zweiten Bestandteil, umfassend Plasma, aufzunehmen,
einen ersten Filter (18), um eines oder mehrere von Leukozyten, roten Blutkörperchen und Thrombozyten aus dem zweiten Bestandteil zu entfernen,
einen zweiten Behälter (14), um den zweiten Bestandteil aus dem ersten Behälter aufzunehmen,
einen dritten Behälter (50), um den ersten Bestandteil aus dem ersten Behälter (12') aufzunehmen, wobei sich der dritte Behälter (50) in einer Abströmflussrichtung des ersten Behälters befindet,
einen vierten Behälter (42), eine zusätzliche Lösung (38) beinhaltend, wobei sich der vierte Behälter in einer Abströmflussrichtung des dritten Behälters befindet, und
einen zweiten Filter (16), um Leukozyten aus dem ersten Bestandteil zu entfernen, wobei sich der zweite Filter stromabwärts des dritten Behälters befindet.

2. Blutverarbeitungssystem nach Anspruch 1, weiter umfassend ein Schlauchverbindungsstück, das sich zwischen dem dritten Behälter (50) und dem zweiten Filter (16) befindet, und weiter umfassend einen Umleitungsschlauchabzweig (30) zur Umleitung von Flüssigkeitsstrom durch den zweiten Filter (16), wobei der Umleitungsabzweig (30) ein erstes Ende und ein zweites Ende aufweist, wobei das erste Ende mit dem Verbindungsstück in Verbindung ist und das zweite Ende mit dem vierten Behälter in Verbindung ist.

3. Blutverarbeitungssystem nach Anspruch 1, weiter beinhaltend einen Filter, um Leukozyten aus Blut in einer Aufströmflussrichtung des ersten Behälters (12') zu entfernen.

4. Blutverarbeitungssystem nach Anspruch 1, wobei sich der zweite Filter 16 zwischen dem dritten Behälter (50) und dem vierten Behälter (42) befindet.

5. Blutverarbeitungssystem nach Anspruch 1, wobei sich der erste Filter (18) in einer Aufströmflussrichtung des zweiten Behälters (14) befindet.

6. Blutverarbeitungssystem nach Anspruch 1, wobei sich der erste Filter (18) zwischen dem ersten Behälter (12') und dem zweiten Behälter 14 befindet.

7. Blutverarbeitungssystem nach Anspruch 1, wobei sich der erste Filter (18) in Abströmflussrichtung des zweiten Behälters (14) befindet.

8. Blutverarbeitungssystem nach Anspruch 1, weiter beinhaltend einen Transferbehälter (34) in Verbindung mit dem zweiten Behälter (14) in einer Abströmflussrichtung des zweiten Behälters (14).

9. Blutverarbeitungssystem nach Anspruch 8, wobei sich der erste Filter (18) zwischen dem zweiten Behälter (14) und dem Transferbehälter (34) befindet.

10. Blutverarbeitungssystem nach Anspruch 6, wobei der erste Filter (18) einen Eingang und einen Ausgang aufweist, wobei das System weiter einen Umleitungsschlauchabzweig (36) zur Umleitung von Flüssigkeitsstrom durch den ersten Filter (18) umfasst, wobei der Umleitungsabzweig (36) ein erstes Ende und ein zweites Ende aufweist, wobei sich das erste Ende zwischen dem ersten Behälter und dem Filtereingang befindet und das zweite Ende sich zwischen dem Filterausgang und dem zweiten Behälter (14) befindet.

11. Blutverarbeitungssystem nach Anspruch 9, wobei der erste Filter (18) einen Eingang und einen Ausgang aufweist, das System weiter umfassend einen Umleitungsschlauchabzweig (36) zur Umleitung von Flüssigkeitsstrom durch den ersten Filter (18), der Umleitungsabzweig (36) ein erstes Ende und ein zweites Ende aufweisend, wobei das erste Ende sich zwischen dem zweiten Behälter und dem Filtereingang befindet und das zweite Ende sich zwischen dem Filterausgang und dem Transferbehälter (34) befindet.

12. Blutverarbeitungssystem nach Anspruch 1, wobei der erste Behälter (12') weiter einen oberen Ausgangsanschluss (56) und einen unteren Ausgangsanschluss (58) umfasst.

13. Blutverarbeitungssystem nach Anspruch 12, wobei der zweite Behälter (14) einen Eingangsanschluss umfasst, und wobei der obere Ausgangsanschluss (56) des ersten Behälters (12') in Verbindung mit dem zweiten Behältereingangsanschluss ist.

14. Blutverarbeitungssystem nach Anspruch 12, wobei der dritte Behälter (50) einen Eingangsanschluss umfasst, und wobei der untere Ausgangsanschluss (58) des ersten Behälters (12') in Verbindung mit dem dritten Behältereingangsanschluss ist.

15. Blutverarbeitungsverfahren, umfassend die Verarbeitung von Vollblut unter Verwendung eines Systems wie definiert in Anspruch 1.

## Revendications

1. Système de traitement du sang comprenant :
un premier récipient (12') destiné à recevoir du sang pour transformation par centrifugation en un premier composant comprenant les globules rouges et un second composant comprenant le plasma,
un premier filtre (18) pour éliminer un ou plusieurs des leucocytes, globules rouges et plaquettes du second composant,
un deuxième récipient (14) destiné à recevoir le second composant du premier récipient,
un troisième récipient (50) destiné à recevoir le premier composant du premier récipient (12'), ledit troisième récipient (50) se trouvant dans une direction d'écoulement aval par rapport audit premier récipient,
un quatrième récipient (42) comprenant une solution additive (38), ledit quatrième récipient se trouvant dans une direction d'écoulement aval par rapport audit troisième récipient, et
un second filtre (16) pour éliminer les leucocytes du premier composant, dans lequel le second filtre est situé en aval dudit troisième récipient.

2. Système de traitement du sang selon la revendication 1, comprenant en outre un raccord de tubulure situé entre ledit troisième récipient (50) et ledit second filtre (16) et comprenant en outre une branche de tubulure de dérivation (30) afin de dériver l'écoulement de fluide via ledit second filtre (16), ladite branche de dérivation (30) ayant une première extrémité et une seconde extrémité, dans lequel ladite première extrémité est en communication avec ledit raccord, et ladite seconde extrémité est en communication avec ledit quatrième récipient.

3. Système de traitement du sang selon la revendication 1, comprenant en outre un filtre pour éliminer les leucocytes du sang dans une direction d'écoulement amont par rapport au premier récipient (12').

4. Système de traitement du sang selon la revendication 1, dans lequel ledit second filtre (16) est situé entre ledit troisième récipient (50) et ledit quatrième récipient (42).

5. Système de traitement du sang selon la revendication 1, dans lequel ledit premier filtre (18) est situé dans une direction d'écoulement amont par rapport au deuxième récipient (14).

6. Système de traitement du sang selon la revendication 1, dans lequel le premier filtre (18) est situé entre le premier récipient 12' et le deuxième récipient (14).

7. Système de traitement du sang selon la revendication 1, dans lequel le premier filtre (18) est situé dans une direction d'écoulement aval par rapport au deuxième récipient 14.

8. Système de traitement du sang selon la revendication 1, comprenant en outre un récipient de transfert (34) communiquant avec le deuxième récipient (14) dans une direction d'écoulement aval par rapport au deuxième récipient (14).

9. Système de traitement du sang selon la revendication 8, dans lequel le premier filtre (18) est situé entre le deuxième récipient (14) et le récipient de transfert (34).

10. Système de traitement du sang selon la revendication 6, dans lequel le premier filtre (18) possède une entrée et une sortie, ledit système comprenant en outre une branche de tubulure de dérivation (36) pour dériver l'écoulement de fluide via ledit premier filtre (18), ladite branche de dérivation (36) ayant une première extrémité et une seconde extrémité, dans lequel ladite première extrémité est située entre ledit premier récipient et ladite entrée de filtre, et ladite seconde extrémité est située entre ladite sortie de filtre et ledit deuxième récipient (14).

11. Système de traitement du sang selon la revendication 9, dans lequel ledit premier filtre (18) possède une entrée et une sortie, ledit système comprenant en outre une branche de tubulure de dérivation (36) pour dériver l'écoulement de fluide via ledit premier filtre (18), ladite branche de dérivation (36) ayant une première extrémité et une seconde extrémité, dans lequel ladite première extrémité est située entre ledit deuxième récipient et ladite entrée de filtre, et ladite seconde extrémité est située entre ladite sortie de filtre et ledit récipient de transfert (34).

12. Système de traitement du sang selon la revendication 1, dans lequel ledit premier récipient (12') comprend en outre un orifice d'entrée supérieur (56) et un orifice de sortie inférieur (58).

13. Système de traitement du sang selon la revendication 12, dans lequel ledit deuxième récipient (14) comprend un orifice d'entrée, et dans lequel ledit orifice de sortie supérieur (56) dudit premier récipient (12') est en communication avec ledit orifice d'entrée du deuxième récipient.

14. Système de traitement du sang selon la revendication 12, dans lequel ledit troisième récipient (50) comprend un orifice d'entrée, et dans lequel ledit orifice de sortie inférieur (58) dudit premier récipient (12') est en communication avec ledit orifice d'entrée du troisième récipient.

15. Système de traitement du sang comprenant le traitement de sang total en utilisant un système selon la revendication 1.
